# EUROPÄISCHE PATENTSCHRIFT

(11) **EP 0 612 349 B1**
(45) Veröffentlichungstag und Bekanntmachung des Hinweises auf die Patenterteilung: **05.03.1997**
(21) Anmeldenummer: 92922434.3
(22) Anmeldetag: 27.10.1992
(51) Int. Cl.: C12N 15/15, C07K 14/00, A61K 38/55

(54) **NEUES THROMBININHIBITORISCHES PROTEIN AUS RAUBWANZEN**
NOVEL THROMBIN INHIBITOR PROTEIN FROM REDUVII
NOUVELLE PROTEINE INHIBITRICE DE LA TROMBINE ISOLEE DANS LES REDUVES

(30) Priorität: 06.11.1991 DE 4136513
(43) Veröffentlichungstag der Anmeldung: 31.08.1994
(73) Patentinhaber: BASF Aktiengesellschaft, 67063 Ludwigshafen (DE)
(72) Erfinder: FRIEDRICH, Thomas, D-6100 Darmstadt (DE); BIALOJAN, Siegfried, D-6836 Oftersheim (DE); KROEGER, Burkhard, D-67117 Limburgerhof (DE); KUENAST, Christoph, D-6701 Otterstadt (DE)
(86) Internationale Anmeldenummer: EP9202450
(87) Internationale Veröffentlichungsnummer: WO9309232

(56) Entgegenhaltungen:
- GB-A- 1 092 421

### SEQUENCE LISTING

### (1) GENERAL INFORMATION:

(i) APPLICANT:
   (A) NAME: BASF Aktiengesellschaft
   (B) STREET: Carl-Bosch-Strasse 38
   (C) CITY: Ludwigshafen
   (E) COUNTRY: Bundesrepublik Deutschland
   (F) POSTAL CODE (ZIP): D-6700
   (G) TELEPHONE: 0621/6048526
   (H) TELEFAX: 0621/6043123
   (I) TELEX: 1762175170
(ii) TITLE OF INVENTION: Neue thrombininhibitorische Proteine aus Raubwanzen
(iii) NUMBER OF SEQUENCES: 22
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25 (EPO)
(v) CURRENT APPLICATION DATA: APPLICATION NUMBER:

### (2) INFORMATION FOR SEQ ID NO:1:

(i) SEQUENCE CHARACTERISTICS:
   (A) LENGTH: 19 amino acids
   (B) TYPE: amino acid
   (D) TOPOLOGY: linear
(ii) MOLECULE TYPE: protein
(iii) HYPOTHETICAL: NO
(iv) ANTI-SENSE: NO
(v) FRAGMENT TYPE: N-terminal
(vi) ORIGINAL SOURCE:
   (A) ORGANISM: Rhodnius prolixus
   (D) DEVELOPMENTAL STAGE: adult
   (F) TISSUE TYPE: whole animals
(ix) FEATURE:
   (A) NAME/KEY: Peptide
   (B) LOCATION: 1..19
(xi) SEQUENCE DESCRIPTION: SEQ ID NO:1:
   Glu Gly Gly Glu Pro Cys Ala Cys Pro His Ala Leu His Arg Val Cys
   1               5                   10                  15
   Gly Ser Asp

### (2) INFORMATION FOR SEQ ID NO:2:

(i) SEQUENCE CHARACTERISTICS:
   (A) LENGTH: 350 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: NO
(iv) ANTI-SENSE: NO
(vi) ORIGINAL SOURCE:
   (A) ORGANISM: Rhodnius prolixus
   (D) DEVELOPMENTAL STAGE: adult
   (F) TISSUE TYPE: whole animals
(vii) IMMEDIATE SOURCE:
   (B) CLONE: pBSKS-/RPTI#5.6
(ix) FEATURE:
   (A) NAME/KEY: CDS
   (B) LOCATION: 1..309
(xi) SEQUENCE DESCRIPTION: SEQ ID NO:2:
   GAA GGT GGT GAA CCG TGC GCT TGC CCC CAC GCT CTG CAT AGA GTT TGC      48
   Glu Gly Gly Glu Pro Cys Ala Cys Pro His Ala Leu His Arg Val Cys
     1               5                  10                  15
   GGC TCT GAT GGT GAA ACT TAT AGC AAC CCT TGT ACG CTG AAC TGT GCT      96
   Gly Ser Asp Gly Glu Thr Tyr Ser Asn Pro Cys Thr Leu Asn Cys Ala
                20                  25                  30
   AAA TTC AAT GGA AAG CCA GAA CTT GTA AAA GTC CAT GAT GGT CCT TGC     144
   Lys Phe Asn Gly Lys Pro Glu Leu Val Lys Val His Asp Gly Pro Cys
            35                  40                  45
   GAA CCG GAT GAG GAT GAA GAT GTT TGC CAA GAA TGT GAT GGT GAT GAA     192
   Glu Pro Asp Glu Asp Glu Asp Val Cys Gln Glu Cys Asp Gly Asp Glu
        50                  55                  60
   TAC AAA CCA GTT TGC GGA TCT GAC GAC ATA ACT TAC GAT AAC AAC TGT     240
   Tyr Lys Pro Val Cys Gly Ser Asp Asp Ile Thr Tyr Asp Asn Asn Cys
    65                  70                  75                  80
   CGA CTA GAG TGT GCC TCT ATC TCT TCC AGC CCA GGA GTT GAA CTG AAA     288
   Arg Leu Glu Cys Ala Ser Ile Ser Ser Ser Pro Gly Val Glu Leu Lys
                    85                  90                  95
   CAT GAA GGA CCT TGT AGA ACC GAAAAAAAAA AAAAAAAAAA AGGTCGCTTC        339
   His Glu Gly Pro Cys Arg Thr
               100
   CGTCGACCAT C                                                        350

### (2) INFORMATION FOR SEQ ID NO:3:

(i) SEQUENCE CHARACTERISTICS:
   (A) LENGTH: 103 amino acids
   (B) TYPE: amino acid
   (D) TOPOLOGY: linear
(ii) MOLECULE TYPE: protein
(xi) SEQUENCE DESCRIPTION: SEQ ID NO:3:
   Glu Gly Gly Glu Pro Cys Ala Cys Pro His Ala Leu His Arg Val Cys
     1               5                  10                  15
   Gly Ser Asp Gly Glu Thr Tyr Ser Asn Pro Cys Thr Leu Asn Cys Ala
                20                  25                  30
   Lys Phe Asn Gly Lys Pro Glu Leu Val Lys Val His Asp Gly Pro Cys
            35                  40                  45
   Glu Pro Asp Glu Asp Glu Asp Val Cys Gln Glu Cys Asp Gly Asp Glu
        50                  55                  60
   Tyr Lys Pro Val Cys Gly Ser Asp Asp Ile Thr Tyr Asp Asn Asn Cys
    65                  70                  75                  80
   Arg Leu Glu Cys Ala Ser Ile Ser Ser Ser Pro Gly Val Glu Leu Lys
                    85                  90                  95
   His Glu Gly Pro Cys Arg Thr
                100

### (2) INFORMATION FOR SEQ ID NO:4:

(i) SEQUENCE CHARACTERISTICS:
   (A) LENGTH: 447 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: NO
(iv) ANTI-SENSE: NO
(vi) ORIGINAL SOURCE:
   (A) ORGANISM: Rhodnius prolixus
   (D) DEVELOPMENTAL STAGE: adult
   (F) TISSUE TYPE: whole animals
(vii) IMMEDIATE SOURCE:
   (A) LIBRARY: R.prolixus uniZAP/XR
   (B) CLONE: uniZAP#3
(ix) FEATURE:
   (A) NAME/KEY: CDS
   (B) LOCATION: 3..275
(ix) FEATURE:
   (A) NAME/KEY: 3'UTR
   (B) LOCATION: 276..447
(xi) SEQUENCE DESCRIPTION: SEQ ID NO:4:
   AA ACT TAT AGC AAC CCT TGT ACG CTG AAC TGT GCT AAA CAC AAT GGA         47
      Thr Tyr Ser Asn Pro Cys Thr Leu Asn Cys Ala Lys His Asn Gly
        1               5                  10                  15
   AAG CCA GGT CTT GTA AAA GTC CAT GAT GGT CCT TGC GAA CCG GAT GAG        95
   Lys Pro Gly Leu Val Lys Val His Asp Gly Pro Cys Glu Pro Asp Glu
                    20                  25                  30
   GAT GAA GAT GTT TGC CAA GAA TGT GAT GAT GTC GAT TAC GAA CCA GTT       143
   Asp Glu Asp Val Cys Gln Glu Cys Asp Asp Val Asp Tyr Glu Pro Val
                35                  40                  45
   TGT GGA ACT GAC GAC AAA ACT TAC GAT AAC AAC TGT CGA CTA GAG TGT       191
   Cys Gly Thr Asp Asp Lys Thr Tyr Asp Asn Asn Cys Arg Leu Glu Cys
            50                  55                  60
   GCC TCT ATC TCT TCC AGC CCA GGA CTT GAA CTG AAG CAC ACA GGA AAA       239
   Ala Ser Ile Ser Ser Ser Pro Gly Leu Glu Leu Lys His Thr Gly Lys
        65                  70                  75
   TGT CTA CCC CAT TTG GAT TTT CCC GAC CCA GTT TAAAGCTTGC ACATAACGGA     292
   Cys Leu Pro His Leu Asp Phe Pro Asp Pro Val
    80                  85                  90
   AAATGCACTA TAGCAGAGTT ATATCACGGT TTATTGTAAA AAAAGATTAT ATGAATTTAT     352
   CATAATATCA ATAAAATAGC TTATTTTAAA AATATTGAAC CAATTTAAAT TTTCAACATA     412
   TGTATATGTA AATAAATTTA AAAAAAAAAA AAAAA                                447

### (2) INFORMATION FOR SEQ ID NO:5:

(i) SEQUENCE CHARACTERISTICS:
   (A) LENGTH: 90 amino acids
   (B) TYPE: amino acid
   (D) TOPOLOGY: linear
(ii) MOLECULE TYPE: protein
(xi) SEQUENCE DESCRIPTION: SEQ ID NO:5:
   Thr Tyr Ser Asn Pro Cys Thr Leu Asn Cys Ala Lys His Asn Gly Lys
    1               5                  10                  15
   Pro Gly Leu Val Lys Val His Asp Gly Pro Cys Glu Pro Asp Glu Asp
               20                  25                  30
   Glu Asp Val Cys Gln Glu Cys Asp Asp Val Asp Tyr Glu Pro Val Cys
           35                  40                  45
   Gly Thr Asp Asp Lys Thr Tyr Asp Asn Asn Cys Arg Leu Glu Cys Ala
       50                  55                  60
   Ser Ile Ser Ser Ser Pro Gly Leu Glu Leu Lys His Thr Gly Lys Cys
    65                  70                  75                  80
   Leu Pro His Leu Asp Phe Pro Asp Pro Val
                   85                  90

### (2) INFORMATION FOR SEQ ID NO:6:

(i) SEQUENCE CHARACTERISTICS:
   (A) LENGTH: 732 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: NO
(iv) ANTI-SENSE: NO
(vi) ORIGINAL SOURCE:
   (A) ORGANISM: Rhodnius prolixus
   (D) DEVELOPMENTAL STAGE: adult
   (F) TISSUE TYPE: whole animals
(vii) IMMEDIATE SOURCE:
   (A) LIBRARY: R.prolixus uniZAP/XR
   (B) CLONE: uniZAP#3.2
(ix) FEATURE:
   (A) NAME/KEY: CDS
   (B) LOCATION: 3..557
(ix) FEATURE:
   (A) NAME/KEY: sig_peptide
   (B) LOCATION: 3..44
(ix) FEATURE:
   (A) NAME/KEY: mat_peptide
   (B) LOCATION: 45..557
(ix) FEATURE:
   (A) NAME/KEY: 3'UTR
   (B) LOCATION: 558..732
(xi) SEQUENCE DESCRIPTION: SEQ ID NO:6:
   AG CGT CTA CTG TTG TTA CTC GGA TTG GCT GCA CTC GTT GCA GCT GAA        47
      Arg Leu Leu Leu Leu Leu Gly Leu Ala Ala Leu Val Ala Ala Glu
      -14             -10                 -5                  1
   GGG GGG GAA CCA TGC GCA TGT CCA CAT GCT CTG CAT AGA GTT TGC GGC       95
   Gly Gly Glu Pro Cys Ala Cys Pro His Ala Leu His Arg Val Cys Gly
                   5                   10                  15
   TCT GAT GGT GAA ACT TAT AGC AAC CCT TGT ACG CTG AAC TGT GCT AAA      143
   Ser Asp Gly Glu Thr Tyr Ser Asn Pro Cys Thr Leu Asn Cys Ala Lys
               20                  25                  30
   TTC AAT GGC AAG CCA GAA CTT GTA AAA GTC CAT GAT GGT CCT TGC GAA      191
   Phe Asn Gly Lys Pro Glu Leu Val Lys Val His Asp Gly Pro Cys Glu
       35                  40                  45
   CCG GAT GAG GAT GAA GAT GTT TGC CAA GAA TGT GAT GGT GAT GAA TAC      239
   Pro Asp Glu Asp Glu Asp Val Cys Gln Glu Cys Asp Gly Asp Glu Tyr
   50                  55                  60                  65
   AAA CCA GTT TGC GGA TCT GAC GGC ATA ACT TAC GAT AAC AAC TGT CGA      287
   Lys Pro Val Cys Gly Ser Asp Gly Ile Thr Tyr Asp Asn Asn Cys Arg
                       70                  75                  80
   CTA GAG TGT GCC TCT ATC TCT TCC AGC CCA GGA GTT GAA CTG AAA CAT      335
   Leu Glu Cys Ala Ser Ile Ser Ser Ser Pro Gly Val Glu Leu Lys His
                   85                  90                  95
   GAA GGG ATA TGT AGA AAG GAG GAA AAG AAA CTT CCT AAA AGA TCT GTG      383
   Glu Gly Ile Cys Arg Lys Glu Glu Lys Lys Leu Pro Lys Arg Ser Val
           100                 105                 110
   GGA TTG GAA CAT ACA TGC GTC TGT CCT TAT AAT TAT TTC CCG GTT TGC      431
   Gly Leu Glu His Thr Cys Val Cys Pro Tyr Asn Tyr Phe Pro Val Cys
       115                 120                 125
   GGA ACA GAT GGG GAA ACC TAT CCC AAC TTG TGC GCC CTC CAA TGT CGT      479
   Gly Thr Asp Gly Glu Thr Tyr Pro Asn Leu Cys Ala Leu Gln Cys Arg
   130                 135                 140                 145
   ATG AGA GAA GTT CCA GGA CTT GAA CTG AAG CAC ACA GGA AAA TGT CTA        527
   Met Arg Glu Val Pro Gly Leu Glu Leu Lys His Thr Gly Lys Cys Leu
                   150                 155                 160
   CCC CAT TTG GAT TTT CCC GAC CCA GTT TAAAGCTTGC ACATAACGGA              574
   Pro His Leu Asp Phe Pro Asp Pro Val
               165                 170
   AAATGCACTA TAGCAGAGTT ATATCACGGT TTATTGTAAA AAAAGATTAT ATGAATTTAT      634
   CATAATATCA ATAAAATAGC TTATTTTAAA AATATTGAAC CAATTTAAAT TTTCAACATA      694
   TGTATATGTA AATAAATTTA AAAAAAAAAA AAAAAAAA                              732

### (2) INFORMATION FOR SEQ ID NO:7:

(i) SEQUENCE CHARACTERISTICS:
   (A) LENGTH: 184 amino acids
   (B) TYPE: amino acid
   (D) TOPOLOGY: linear
(ii) MOLECULE TYPE: protein
(xi) SEQUENCE DESCRIPTION: SEQ ID NO:7:
   Arg Leu Leu Leu Leu Leu Gly Leu Ala Ala Leu Val Ala Ala Glu Gly
   -14             -10                     -5                   1
   Gly Glu Pro Cys Ala Cys Pro His Ala Leu His Arg Val Cys Gly Ser
                5                  10                  15
   Asp Gly Glu Thr Tyr Ser Asn Pro Cys Thr Leu Asn Cys Ala Lys Phe
        20                  25                  30
   Asn Gly Lys Pro Glu Leu Val Lys Val His Asp Gly Pro Cys Glu Pro
    35                  40                  45                  50
   Asp Glu Asp Glu Asp Val Cys Gln Glu Cys Asp Gly Asp Glu Tyr Lys
                    55                  60                  65
   Pro Val Cys Gly Ser Asp Gly Ile Thr Tyr Asp Asn Asn Cys Arg Leu
                70                  75                  80
   Glu Cys Ala Ser Ile Ser Ser Ser Pro Gly Val Glu Leu Lys His Glu
            85                  90                  95
   Gly Ile Cys Arg Lys Glu Glu Lys Lys Leu Pro Lys Arg Ser Val Gly
       100                 105                 110
   Leu Glu His Thr Cys Val Cys Pro Tyr Asn Tyr Phe Pro Val Cys Gly
   115                 120                 125                 130
   Thr Asp Gly Glu Thr Tyr Pro Asn Leu Cys Ala Leu Gln Cys Arg Met
                   135                 140                 145
   Arg Glu Val Pro Gly Leu Glu Leu Lys His Thr Gly Lys Cys Leu Pro
               150                 155                 160
   His Leu Asp Phe Pro Asp Pro Val
           165                 170

### (2) INFORMATION FOR SEQ ID NO:8:

(i) SEQUENCE CHARACTERISTICS:
   (A) LENGTH: 677 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: NO
(iv) ANTI-SENSE: NO
(vi) ORIGINAL SOURCE:
   (A) ORGANISM: Rhodnius prolixus
   (D) DEVELOPMENTAL STAGE: adult
   (F) TISSUE TYPE: whole animals
(vii) IMMEDIATE SOURCE:
   (A) LIBRARY: R.prolixus uniZAP/XR
   (B) CLONE: uniZAP#5
(ix) FEATURE:
   (A) NAME/KEY: CDS
   (B) LOCATION: 1..561
(ix) FEATURE:
   (A) NAME/KEY: 3'UTR
   (B) LOCATION: 562..677
(ix) FEATURE:
   (A) NAME/KEY: sig_peptide
   (B) LOCATION: 1..48
(ix) FEATURE:
   (A) NAME/KEY: mat_peptide
   (B) LOCATION: 49..561
(xi) SEQUENCE DESCRIPTION: SEQ ID NO:8:
   ATG AAG CGT CTA CTG TTG TTA CTC GGA TTG GCT GCA CTC GTT GCA GCT          48
   Met Lys Arg Leu Leu Leu Leu Leu Gly Leu Ala Ala Leu Val Ala Ala
   -16 -15                 -10                     -5
   GAA GGG GGG GAA CCA TGC GCA TGT CCA CAT GCT CTG CAT AGA GTT TGC        96
   Glu Gly Gly Glu Pro Cys Ala Cys Pro His Ala Leu His Arg Val Cys
    1               5                  10                  15
   GGC TCT GAT GGT GAA ACT TAT AGC AAC CCT TGT ACG CTG AAC TGT GCT       144
   Gly Ser Asp Gly Glu Thr Tyr Ser Asn Pro Cys Thr Leu Asn Cys Ala
               20                  25                  30
   AAA TTT AAT GGA AAG CCA GAA CTT GTA AAA GTC CAT GAT GGT CCT TGC       192
   Lys Phe Asn Gly Lys Pro Glu Leu Val Lys Val His Asp Gly Pro Cys
           35                  40                  45
   GAA CCG GAT GAG GAT GAA GAT GTT TGC CAA GAA TGT GAT GGT GAT GAA       240
   Glu Pro Asp Glu Asp Glu Asp Val Cys Gln Glu Cys Asp Gly Asp Glu
       50                  55                  60
   TAC AAA CCA GTT TGC GGA TCT GAC GGC ATA ACT TAC GAT AAC AAC TGT       288
   Tyr Lys Pro Val Cys Gly Ser Asp Gly Ile Thr Tyr Asp Asn Asn Cys
   65                  70                  75                  80
   CGA CTA GAG TGT GCC TCT ATC TCT TCC AGC CCA GGA GTT GAA CTG AAA       336
   Arg Leu Glu Cys Ala Ser Ile Ser Ser Ser Pro Gly Val Glu Leu Lys
                   85                  90                  95
   CAT GAA GGG ATA TGT AGA AAG GAG GAA AAG AAA CTT CCT AAA AGA TCT       384
   His Glu Gly Ile Cys Arg Lys Glu Glu Lys Lys Leu Pro Lys Arg Ser
               100                 105                 110
   GTG GGA TTG GAA CAT ACA TGC GTC TGT CCT TAT AAT TAT TTC CCG GTT       432
   Val Gly Leu Glu His Thr Cys Val Cys Pro Tyr Asn Tyr Phe Pro Val
           115                 120                 125
   TGC GGA ACA GAT GGG GAA ACC TAT CCC AAC TTG TGC GCC CTC CAA TGT       480
   Cys Gly Thr Asp Gly Glu Thr Tyr Pro Asn Leu Cys Ala Leu Gln Cys
       130                 135                 140
   CGT ATG AGA GAA GTT CCA GGA CTT GAA CTG AAG CAC ACA GGA AAA TGT       528
   Arg Met Arg Glu Val Pro Gly Leu Glu Leu Lys His Thr Gly Lys Cys
   145                 150                 155                 160
   CTA CCC CAT TTG GAT TTT CCC GAC CCA GTT TAAAGCTTGC ACATAACGGA         578
   Leu Pro His Leu Asp Phe Pro Asp Pro Val
                   165                 170
   AAATGCACTA TAGCAGAGTT ATATCACGGT TTATTGTAAA AAAAGGATTA TATGAATTTA     638
   TCATAATATC AATAAAATAG CTTATTTTAA AAATATTGA                            677

### (2) INFORMATION FOR SEQ ID NO:9:

(i) SEQUENCE CHARACTERISTICS:
   (A) LENGTH: 186 amino acids
   (B) TYPE: amino acid
   (D) TOPOLOGY: linear
(ii) MOLECULE TYPE: protein
(xi) SEQUENCE DESCRIPTION: SEQ ID NO:9:
   Met Lys Arg Leu Leu Leu Leu Leu Gly Leu Ala Ala Leu Val Ala Ala
   -16 -15                 -10                     -5
   Glu Gly Gly Glu Pro Cys Ala Cys Pro His Ala Leu His Arg Val Cys
     1               5                  10                  15
   Gly Ser Asp Gly Glu Thr Tyr Ser Asn Pro Cys Thr Leu Asn Cys Ala
                20                  25                  30
   Lys Phe Asn Gly Lys Pro Glu Leu Val Lys Val His Asp Gly Pro Cys
            35                  40                  45
   Glu Pro Asp Glu Asp Glu Asp Val Cys Gln Glu Cys Asp Gly Asp Glu
       50                  55                  60
   Tyr Lys Pro Val Cys Gly Ser Asp Gly Ile Thr Tyr Asp Asn Asn Cys
    65                  70                  75                  80
   Arg Leu Glu Cys Ala Ser Ile Ser Ser Ser Pro Gly Val Glu Leu Lys
                    85                  90                  95
   His Glu Gly Ile Cys Arg Lys Glu Glu Lys Lys Leu Pro Lys Arg Ser
               100                 105                 110
   Val Gly Leu Glu His Thr Cys Val Cys Pro Tyr Asn Tyr Phe Pro Val
           115                 120                 125
   Cys Gly Thr Asp Gly Glu Thr Tyr Pro Asn Leu Cys Ala Leu Gln Cys
       130                 135                 140
   Arg Met Arg Glu Val Pro Gly Leu Glu Leu Lys His Thr Gly Lys Cys
   145                 150                 155                 160
   Leu Pro His Leu Asp Phe Pro Asp Pro Val
                   165                 170

### (2) INFORMATION FOR SEQ ID NO:10:

(i) SEQUENCE CHARACTERISTICS:
   (A) LENGTH: 566 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: NO
(iv) ANTI-SENSE: NO
(vi) ORIGINAL SOURCE:
   (A) ORGANISM: Rhodnius prolixus
   (D) DEVELOPMENTAL STAGE: adult
   (F) TISSUE TYPE: whole animals
(vii) IMMEDIATE SOURCE:
   (A) LIBRARY: R.prolixus uniZAP/XR
   (B) CLONE: uniZAP#8
(ix) FEATURE:
   (A) NAME/KEY: CDS
   (B) LOCATION: 2..391
(ix) FEATURE:
   (A) NAME/KEY: 3'UTR
   (B) LOCATION: 392..566
(xi) SEQUENCE DESCRIPTION: SEQ ID NO:10:
   A AAA GTC CAT GAT GGT CCT TGC GAA CCG GAT GAG GAT GAA GAT GTT        46
     Lys Val His Asp Gly Pro Cys Glu Pro Asp Glu Asp Glu Asp Val
       1               5                  10                  15
   TGC CAA GAA TGT GAT GAT GTC GAT TAC GAA CCA GTT TGT GGA ACT GAC      94
   Cys Gln Glu Cys Asp Asp Val Asp Tyr Glu Pro Val Cys Gly Thr Asp
                    20                  25                  30
   GAC AAA ACT TAC GAT AAC AAC TGT CGA CTA GAG TGT GCC TCT ATC TCT     142
   Asp Lys Thr Tyr Asp Asn Asn Cys Arg Leu Glu Cys Ala Ser Ile Ser
                35                  40                  45
   TCC AGC CCA GGA GTT GAA CTG AAA CAT GAA GGG ATA TGT AGA AAG GAG     190
   Ser Ser Pro Gly Val Glu Leu Lys His Glu Gly Ile Cys Arg Lys Glu
            50                  55                  60
   GAA AAG AAA CTT CCT AAA AGA TCT GTG GGA TTG GAA CAT ACA TGC GTC     238
   Glu Lys Lys Leu Pro Lys Arg Ser Val Gly Leu Glu His Thr Cys Val
        65                  70                  75
   TGT CCT TAT AAT TAT TTC CCG GTT TGC GGA ACA GAT GGG GAA ACC TAT     286
   Cys Pro Tyr Asn Tyr Phe Pro Val Cys Gly Thr Asp Gly Glu Thr Tyr
    80                  85                  90                  95
   CCC AAC TTG TGC GCC CTC CAA TGT CGT ATG AGA GAA GTT CCA GGA CTT     334
   Pro Asn Leu Cys Ala Leu Gln Cys Arg Met Arg Glu Val Pro Gly Leu
                   100                 105                 110
   GAA CTG AAG CAC ACA GGA AAA TGT CTA CCC CAT TTG GAT TTT CCC GAC     382
   Glu Leu Lys His Thr Gly Lys Cys Leu Pro His Leu Asp Phe Pro Asp
               115                 120                 125
   CCA GTT TAAAGCTTGC ACATAACGGA AAATGCACTA TAGCAGAGGT ATATCACGGT       438
   Pro Val
           130
   TTATTGTAAA AAAAGGTTAT ATGAATTTAT CATAATATCA ATTAAAATAG CTTATTTTAA    498
   AAATATTGCC CATTTAAATT TTCAACATAT GTATATGTAA ATAAATTTAA AAAAAAAAAA    558
   AAAAAAAA                                                             566

### (2) INFORMATION FOR SEQ ID NO:11:

(i) SEQUENCE CHARACTERISTICS:
   (A) LENGTH: 129 amino acids
   (B) TYPE: amino acid
   (D) TOPOLOGY: linear
(ii) MOLECULE TYPE: protein
(xi) SEQUENCE DESCRIPTION: SEQ ID NO:11:
   Lys Val His Asp Gly Pro Cys Glu Pro Asp Glu Asp Glu Asp Val Cys
     1               5                  10                  15
   Gln Glu Cys Asp Asp Val Asp Tyr Glu Pro Val Cys Gly Thr Asp Asp
                20                  25                  30
   Lys Thr Tyr Asp Asn Asn Cys Arg Leu Glu Cys Ala Ser Ile Ser Ser
            35                  40                  45
   Ser Pro Gly Val Glu Leu Lys His Glu Gly Ile Cys Arg Lys Glu Glu
        50                  55                  60
   Lys Lys Leu Pro Lys Arg Ser Val Gly Leu Glu His Thr Cys Val Cys
    65                  70                  75                  80
   Pro Tyr Asn Tyr Phe Pro Val Cys Gly Thr Asp Gly Glu Thr Tyr Pro
                    85                  90                  95
   Asn Leu Cys Ala Leu Gln Cys Arg Met Arg Glu Val Pro Gly Leu Glu
               100                 105                 110
   Leu Lys His Thr Gly Lys Cys Leu Pro His Leu Asp Phe Pro Asp Pro
           115                 120                 125
   Val

### (2) INFORMATION FOR SEQ ID NO:12:

(i) SEQUENCE CHARACTERISTICS:
   (A) LENGTH: 327 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: NO
(iv) ANTI-SENSE: NO
(vi) ORIGINAL SOURCE:
   (A) ORGANISM: Rhodnius prolixus
   (D) DEVELOPMENTAL STAGE: adult
   (F) TISSUE TYPE: whole animals
(vii) IMMEDIATE SOURCE:
   (A) LIBRARY: R.prolixus uniZAP/XR
   (B) CLONE: uniZAP#11
(ix) FEATURE:
   (A) NAME/KEY: CDS
   (B) LOCATION: 1..327
(xi) SEQUENCE DESCRIPTION: SEQ ID NO:12:

### (2) INFORMATION FOR SEQ ID NO:13:

(i) SEQUENCE CHARACTERISTICS:
   (A) LENGTH: 108 amino acids
   (B) TYPE: amino acid
   (D) TOPOLOGY: linear
(ii) MOLECULE TYPE: protein
(xi) SEQUENCE DESCRIPTION: SEQ ID NO:13:
   Val Asp Tyr Glu Pro Val Cys Gly Thr Asp Asp Lys Thr Tyr Asp Asn
    1               5                  10                  15
   Asn Cys Arg Leu Glu Cys Ala Ser Ile Ser Ser Ser Pro Gly Val Glu
               20                  25                  30
   Leu Lys His Glu Gly Ile Cys Arg Lys Glu Glu Lys Lys Leu Pro Lys
           35                  40                  45
   Arg Ser Val Gly Leu Glu His Thr Cys Val Cys Pro Tyr Asn Tyr Phe
       50                  55                  60
   Pro Val Cys Gly Thr Asp Gly Glu Thr Tyr Pro Asn Leu Cys Gly Leu
   65                  70                  75                  80
   Gln Cys Arg Met Arg Glu Val Pro Gly Leu Glu Leu Lys His Thr Gly
                   85                  90                  95
   Lys Cys Leu Pro His Leu Asp Phe Pro Asp Pro Val
               100                 105

### (2) INFORMATION FOR SEQ ID NO:14:

(i) SEQUENCE CHARACTERISTICS:
   (A) LENGTH: 616 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: NO
(iv) ANTI-SENSE: NO
(vi) ORIGINAL SOURCE:
   (A) ORGANISM: Rhodnius prolixus
   (D) DEVELOPMENTAL STAGE: adult
   (F) TISSUE TYPE: whole animals
(vii) IMMEDIATE SOURCE:
   (A) LIBRARY: R.prolixus uniZAP/XR
   (B) CLONE: uniZAP#12
(ix) FEATURE:
   (A) NAME/KEY: CDS
   (B) LOCATION: 2..556
(ix) FEATURE:
   (A) NAME/KEY: 3'UTR
   (B) LOCATION: 557..616
(ix) FEATURE:
   (A) NAME/KEY: sig_peptide
   (B) LOCATION: 2..43
(ix) FEATURE:
   (A) NAME/KEY: mat_peptide
   (B) LOCATION: 44..556
(xi) SEQUENCE DESCRIPTION: SEQ ID NO:14:
   G CGT CTA CTG TTG TTA CTC GGA TTG GCT GCA CTC GTT GCA GCT GAA      46
     Arg Leu Leu Leu Leu Leu Gly Leu Ala Ala Leu Val Ala Ala Glu
     -14             -10                 -5                   1
   GGG GGG GAA CCA TGC GCA TGT CCA CAT GCT CTG CAT AGA GTT TGC GGC    94
   Gly Gly Glu Pro Cys Ala Cys Pro His Ala Leu His Arg Val Cys Gly
                   5                  10                  15
   TCT GAT GGT GAA ACT TAT AGC AAC CCT TGT ACG CTG AAC TGT GCT AAA   142
   Ser Asp Gly Glu Thr Tyr Ser Asn Pro Cys Thr Leu Asn Cys Ala Lys
           20                  25                  30
   TTC AAT GGA AAG CCA GAA CTT GTA AAA GTC CAT GAT GGT CCT TGC GAA   190
   Phe Asn Gly Lys Pro Glu Leu Val Lys Val His Asp Gly Pro Cys Glu
       35                  40                  45
   CCG GAT GAG GAT GAA GAT GTT TGC CAA GAA TGT GAT GAT GTC GAT TAC   238
   Pro Asp Glu Asp Glu Asp Val Cys Gln Glu Cys Asp Asp Val Asp Tyr
   50                  55                  60                  65
   GAA CCA GTT TGT GGA ACT GAC GAC AAA ACT TAC GAT AAC AAC TGT CGA   286
   Glu Pro Val Cys Gly Thr Asp Asp Lys Thr Tyr Asp Asn Asn Cys Arg
                   70                  75                  80
   CTA GAG TGT GCC TCT ATC TCT TCC AGC CCA GGA GTT GAA CTG AAA CAT   334
   Leu Glu Cys Ala Ser Ile Ser Ser Ser Pro Gly Val Glu Leu Lys His
               85                  90                  95
   GAA GGG ATA TGT AGA AAG GAG GAA AAG AAA CTT CCT AAA AGA TCT GTG   382
   Glu Gly Ile Cys Arg Lys Glu Glu Lys Lys Leu Pro Lys Arg Ser Val
           100                 105                 110
   GGA TTG GAA CAT ACA TGC GTC TGT CCT TAT AAT TAT TTC CCG GTT TGC   430
   Gly Leu Glu His Thr Cys Val Cys Pro Tyr Asn Tyr Phe Pro Val Cys
       115                 120                 125
   GGA ACA GAT GGG GAA ACC TAT CCC AAC TTG TGC GCC CTC CAA TGT CGT      478
   Gly Thr Asp Gly Glu Thr Tyr Pro Asn Leu Cys Ala Leu Gln Cys Arg
   130                 135                 140                 145
   ATG AGA GAA GTT CCA GGA CTT GAA CTG AAG CAC ACA GGA AAA TGT CTA      526
   Met Arg Glu Val Pro Gly Leu Glu Leu Lys His Thr Gly Lys Cys Leu
                   150                 155                 160
   CCC CAT TTG GAT TTT CCC GAC CCA GTT TAAAGCTTGC ACATAACGGA            573
   Pro His Leu Asp Phe Pro Asp Pro Val
               165                 170
   AAATGCACTA TAGCAGAGTT ATATCACGGT TTATTGTAAA AAA                      616

### (2) INFORMATION FOR SEQ ID NO:15:

(i) SEQUENCE CHARACTERISTICS:
   (A) LENGTH: 184 amino acids
   (B) TYPE: amino acid
   (D) TOPOLOGY: linear
(ii) MOLECULE TYPE: protein
(xi) SEQUENCE DESCRIPTION: SEQ ID NO:15:
   Arg Leu Leu Leu Leu Leu Gly Leu Ala Ala Leu Val Ala Ala Glu Gly
   -14             -10                     -5                   1
   Gly Glu Pro Cys Ala Cys Pro His Ala Leu His Arg Val Cys Gly Ser
               5                   10                  15
   Asp Gly Glu Thr Tyr Ser Asn Pro Cys Thr Leu Asn Cys Ala Lys Phe
       20                  25                  30
   Asn Gly Lys Pro Glu Leu Val Lys Val His Asp Gly Pro Cys Glu Pro
   35                  40                  45                  50
   Asp Glu Asp Glu Asp Val Cys Gln Glu Cys Asp Asp Val Asp Tyr Glu
                   55                  60                  65
   Pro Val Cys Gly Thr Asp Asp Lys Thr Tyr Asp Asn Asn Cys Arg Leu
               70                  75                  80
   Glu Cys Ala Ser Ile Ser Ser Ser Pro Gly Val Glu Leu Lys His Glu
           85                  90                  95
   Gly Ile Cys Arg Lys Glu Glu Lys Lys Leu Pro Lys Arg Ser Val Gly
       100                 105                 110
   Leu Glu His Thr Cys Val Cys Pro Tyr Asn Tyr Phe Pro Val Cys Gly
   115                 120                 125                 130
   Thr Asp Gly Glu Thr Tyr Pro Asn Leu Cys Ala Leu Gln Cys Arg Met
                   135                 140                 145
   Arg Glu Val Pro Gly Leu Glu Leu Lys His Thr Gly Lys Cys Leu Pro
               150                 155                 160
   His Leu Asp Phe Pro Asp Pro Val
           165                 170

### (2) INFORMATION FOR SEQ ID NO:16:

(i) SEQUENCE CHARACTERISTICS:
   (A) LENGTH: 1282 base pairs
   (B) TYPE: nucleic acid
   (C) STRANDEDNESS: single
   (D) TOPOLOGY: linear
(ii) MOLECULE TYPE: cDNA to mRNA
(iii) HYPOTHETICAL: NO
(iv) ANTI-SENSE: NO
(vi) ORIGINAL SOURCE:
   (A) ORGANISM: Rhodnius prolixus
   (D) DEVELOPMENTAL STAGE: adult
   (F) TISSUE TYPE: whole aqnimals
(vii) IMMEDIATE SOURCE:
   (A) LIBRARY: R.prolixus uniZAP/XR
   (B) CLONE: uniZAP#9
(ix) FEATURE:
   (A) NAME/KEY: CDS
   (B) LOCATION: 3..1109
(ix) FEATURE:
   (A) NAME/KEY: sig_peptide
   (B) LOCATION: 3..44
(ix) FEATURE:
   (A) NAME/KEY: mat_peptide
   (B) LOCATION: 45..1109
(ix) FEATURE:
   (A) NAME/KEY: 3'UTR
   (B) LOCATION: 1110..1282
(xi) SEQUENCE DESCRIPTION: SEQ ID NO:16:
   AG CGT CTA CTG TTG TTA CTC GGA TTG GCT GCA CTC GTT GCA GCT GAA          47
      Arg Leu Leu Leu Leu Leu Gly Leu Ala Ala Leu Val Ala Ala Glu
      -14             -10                     -5                   1
   GGG GGG GAA CCA TGC GCA TGT CCA CAT GCT CTG CAT AGA GTT TGC GGC      95
   Gly Gly Glu Pro Cys Ala Cys Pro His Ala Leu His Arg Val Cys Gly
                     5                  10                  15
   TCT GAT GGT GAA ACT TAT AGC AAC CCT TGT ACG CTG AAC TGT GCT AAA     143
   Ser Asp Gly Glu Thr Tyr Ser Asn Pro Cys Thr Leu Asn Cys Ala Lys
                20                  25                  30
   TTC AAT GGA AAG CCA GAA CTT GTA AAA GTC CAT GAT GGT CCT TGC GAA     191
   Phe Asn Gly Lys Pro Glu Leu Val Lys Val His Asp Gly Pro Cys Glu
            35                  40                  45
   CCG GAT GAG GAT GAA GAT GTT TGC CAA GAA TGT GAT GGT GAT GAA TAC     239
   Pro Asp Glu Asp Glu Asp Val Cys Gln Glu Cys Asp Gly Asp Glu Tyr
    50                  55                  60                  65
   AAA CCA GTT TGC GGA TCT GAC GAC ATA ACT TAC GAT AAC AAC TGT CGA     287
   Lys Pro Val Cys Gly Ser Asp Asp Ile Thr Tyr Asp Asn Asn Cys Arg
                        70                  75                  80
   CTA GAG TGT GCC TCT ATC TCT TCC AGC CCA GGA GTT GAA CTG AAA CAT     335
   Leu Glu Cys Ala Ser Ile Ser Ser Ser Pro Gly Val Glu Leu Lys His
                    85                  90                  95
   GAA GGA CCT TGT AGA ACC GAG GAA AAG AAA ATT CTT AAA AGA TCT GAT     383
   Glu Gly Pro Cys Arg Thr Glu Glu Lys Lys Ile Leu Lys Arg Ser Asp
               100                 105                 110
   GAA TTC GAA ATG TAT AGA TGC GCA TGT CCG AAA ATA TAT TAT CCG GTT     431
   Glu Phe Glu Met Tyr Arg Cys Ala Cys Pro Lys Ile Tyr Tyr Pro Val
           115                 120                 125
   TGC GGA ACA GAT GGT GAA ACC TAT CCC AAC TTG TGC GTC CTC GAA TGT     479
   Cys Gly Thr Asp Gly Glu Thr Tyr Pro Asn Leu Cys Val Leu Glu Cys
   130                 135                 140                 145
   CAT ATG AGA ATG AAT CCA GGA CTT CAA TTG CAC CAT TAT GGA CAT TGT     527
   His Met Arg Met Asn Pro Gly Leu Gln Leu His His Tyr Gly His Cys
                       150                 155                 160
   CAA CAT CAT CAT CAC CAT CAT CCT CCT CCT CAT CAC CAT CAT CAT CAT     575
   Gln His His His His His His Pro Pro Pro His His His His His His
                   165                 170                 175
   CAT CCT CAT CAC ACC ACT GAG AAA CCA GTA GAA CCA TGC GCA TGT CCA     623
   His Pro His His Thr Thr Glu Lys Pro Val Glu Pro Cys Ala Cys Pro
               180                 185                 190
   CAT GCT CTG CAT AGA GTT TGC GGC TCT GAT GGT GAA ACT TAT AGC AAC     671
   His Ala Leu His Arg Val Cys Gly Ser Asp Gly Glu Thr Tyr Ser Asn
           195                 200                 205
   CCT TGT ACG CTG AAC TGT GCT AAA CAC AAT GGA AAG CCA GGT CTT GTA        719
   Pro Cys Thr Leu Asn Cys Ala Lys His Asn Gly Lys Pro Gly Leu Val
   210                 215                 220                 225
   AAA GTC CAT GAT GGT CCT TGC GAA CCG GAT GAG GAT GAA GAT GTT TGC        767
   Lys Val His Asp Gly Pro Cys Glu Pro Asp Glu Asp Glu Asp Val Cys
                   230                 235                 240
   CAA GAA TGT GAT GAT GTC GAT TAC GAA CCA GTT TGT GGA ACT GAC GAC        815
   Gln Glu Cys Asp Asp Val Asp Tyr Glu Pro Val Cys Gly Thr Asp Asp
               245                 250                 255
   AAA ACT TAC GAT AAC AAC TGT CGA CTA GAG TGT GCC TCT ATC TCT TCC        863
   Lys Thr Tyr Asp Asn Asn Cys Arg Leu Glu Cys Ala Ser Ile Ser Ser
           260                 265                 270
   AGC CCA GGA GTT GAA CTG AAA CAT GAA GGG ATA TGT AGA AAG GAG GAA        911
   Ser Pro Gly Val Glu Leu Lys His Glu Gly Ile Cys Arg Lys Glu Glu
       275                 280                 285
   AAG AAA CTT CCT AAA AGA TCT GTG GGA TTG GAA CAT ACA TGC GTC TGT        959
   Lys Lys Leu Pro Lys Arg Ser Val Gly Leu Glu His Thr Cys Val Cys
   290                 295                 300                 305
   CCT TAT AAT TAT TTC CCG GTT TGC GGA ACA GAT GGG GAA ACC TAT CCC       1007
   Pro Tyr Asn Tyr Phe Pro Val Cys Gly Thr Asp Gly Glu Thr Tyr Pro
                   310                 315                 320
   AAC TTG TGC GCC CTC CAA TGC CGT ATG AGA GAA GTT CCA GGA CTT GAA       1055
   Asn Leu Cys Ala Leu Gln Cys Arg Met Arg Glu Val Pro Gly Leu Glu
               325                 330                 335
   CTG AAG CAC ACA GGA AAA TGT CTA CCC CAT TTG GAT TTT CCC GAC CCA       1103
   Leu Lys His Thr Gly Lys Cys Leu Pro His Leu Asp Phe Pro Asp Pro
           340                 345                 350
   GTT TAAAGCTTGC ACATAACGGA AAATGCACTA TAGCAGAGTT ATATCACGGT            1156
   Val
       355
   TTATTGTAAA AAAAGATTAT ATGAATTTAT CATAATATCA ATAAAATAGC TTATTTTAAA     1216
   AATATTGAAC CAATTTAAAT TTTCAACATA TGTATATGTA AATAAATTTA AAAAAAAAAA     1276
   AAAAAA                                                                1282

### (2) INFORMATION FOR SEQ ID NO:17:

(i) SEQUENCE CHARACTERISTICS:
   (A) LENGTH: 368 amino acids
   (B) TYPE: amino acid
   (D) TOPOLOGY: linear
(ii) MOLECULE TYPE: protein
(xi) SEQUENCE DESCRIPTION: SEQ ID NO:17:

### (2) INFORMATION FOR SEQ ID NO:18:

(i) SEQUENCE CHARACTERISTICS:
   (A) LENGTH: 11 amino acids
   (B) TYPE: amino acid
   (D) TOPOLOGY: linear
(ii) MOLECULE TYPE: peptide
(iii) HYPOTHETICAL: NO
(iv) ANTI-SENSE: NO
(v) FRAGMENT TYPE: N-terminal
(vi) ORIGINAL SOURCE:
   (A) ORGANISM: Rhodnius prolixus
   (D) DEVELOPMENTAL STAGE: adult
   (F) TISSUE TYPE: whole animals
(ix) FEATURE:
   (A) NAME/KEY: Peptide
   (B) LOCATION: 1..11
(xi) SEQUENCE DESCRIPTION: SEQ ID NO:18:
   Glu Gly Gly Glu Pro Cys Ala Cys Pro His Ala
   1               5                   10

### (2) INFORMATION FOR SEQ ID NO:19:

(i) SEQUENCE CHARACTERISTICS:
   (A) LENGTH: 32 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: YES
(iv) ANTI-SENSE: NO
(vi) ORIGINAL SOURCE:
   (A) ORGANISM: Rhodnius prolixus
(xi) SEQUENCE DESCRIPTION: SEQ ID NO:19:
   GAAGGTGGTG AACCNTGYGC NTGYCCNCAY GC 32

### (2) INFORMATION FOR SEQ ID NO:20:

(i) SEQUENCE CHARACTERISTICS:
   (A) LENGTH: 45 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: YES
(iv) ANTI-SENSE: NO
(xi) SEQUENCE DESCRIPTION: SEQ ID NO:20:
   CGAGGGGGAT GGTCGACGGA AGCGACCTTT TTTTTTTTTT TTTTT 45

### (2) INFORMATION FOR SEQ ID NO:21:

(i) SEQUENCE CHARACTERISTICS:
   (A) LENGTH: 19 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: YES
(iv) ANTI-SENSE: NO
(xi) SEQUENCE DESCRIPTION: SEQ ID NO:21:
   CGAGGGGGAT GGTCGACGG 19

### (2) INFORMATION FOR SEQ ID NO:22:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: DNA (genomic)
(iii) HYPOTHETICAL: YES
(iv) ANTI-SENSE: NO
(xi) SEQUENCE DESCRIPTION: SEQ ID NO:22:
   GATGGTCGAC GGAAGCGACC                                              20

## Beschreibung

Die vorliegende Erfindung betrifft ein neues thrombininhibitorisches Protein aus Raubwanzen sowie Verfahren zu dessen Herstellung.

Thrombin-Inhibitoren sind wichtige therapeutische Substanzen, die beispielsweise zur Prophylaxe oder Behandlung von Thrombosen oder arteriellen Reokklusionen verwendet werden.

In der deutschen Offenlegungsschrift DE 39 31 839 wird ein Thrombinininhibitor, der aus der Lederzecke Ornithodoros moubata isoliert worden ist, beschrieben. Dieses Protein besitzt ein Molekulargewicht von etwa 15000 Dalton, einen isoelektrischen Punkt von pH 4-5 und die N-terminale Aminosäuresequenz SDYEFPPPKKXRPG.

In der europäischen Offenlegungsschrift EP 345 614 wird der thrombininhibitorische Wirkstoff Amblyommin beschrieben, der aus Schildzecken isoliert wird. Es handelt sich dabei um ein Protein mit einem Molekulargewicht von 20 000 - 30 000 Dalton und einem isoelektrischen Punkt zwischen 5,05 und 5,65.

Bisher wurde jedoch noch kein Protein mit thrombininhibitorischer Wirkung gefunden, das als Arzneimittel hinsichtlich hoher Wirksamkeit, fehlender Antigenizität, langer biologischer Halbwertzeit, geringer Nebenwirkung wie z.B. Blutungsneigung, vorteilhaft geeignet ist.

Der Erfindung lag daher die Aufgabe zugrunde, neue Thrombin-Inhibitoren zur Verfügung zu stellen, die als Arzneimittel hinsichtlich der oben genannten Eigenschaften geeignet sind.

Demgemäß wurde ein neues thrombininhibitorisches Protein aus Raubwanzen isoliert.

Das neue Protein besitzt folgende physikochemischen Eigenschaften. Durch Molekularsiebchromatographie wird ihm ein Molekulargewicht von 20000 - 24000 Dalton zugeordnet. In einem SDS-Polyacrylamidgel wird ein Molekulargewicht von 12000 ± 2000 Dalton bestimmt. Die Bestimmung des isoelektrischen Punktes ergibt einen pH-Wert zwischen 3,7 und 4,7.

Das Protein bindet spezifisch an eine Thrombin-Affinitätssäule. Es hemmt die biologische Aktivität von Thrombin in einem in-vitro Enzymtest.

Folgende N-terminale Aminosäuresequenz wurde von dem Protein bestimmt (SEQ ID NO: 1):

Das erfindungsgemäße Protein enthält eine Aminosäuresequenz von 103 Aminosäuren, die im Sequenzprotokoll SEQ ID NO: 3 angegeben ist.

Die für diese 103 Aminosäuren lange Proteinsequenz codierende DNA ist im Sequenzprotokoll SEQ ID NO: 2 aufgeführt.

Das erfindungsgemäße Protein kann darüber hinaus am C-Terminus noch weitere Aminosäuren enthalten. Ebenso kann das Protein am N-Terminus noch weitere Aminosäuresequenzen wie natürliche oder heterologe Leadersequenzen oder zusätzliche Aminosäuren wie Methionin enthalten.

Aus der im Sequenzprotokoll SEQ ID NO: 3 angegebenen Aminosäuresequenz lassen sich zwei Domänen in diesem Molekül vorhersagen. Die erste Domäne ist im wesentlichen durch die Cysteinreste an den Positionen 6 und 48, die zweite Domäne durch die Cysteinreste an den Positionen 57 und 101 begrenzt.

Weiterhin sind Gegenstand der Erfindung DNA-Sequenzen, die für Proteine mit thrombininhibitorischer Wirkung codieren, und die aus der Gruppe, die von
a) DNA-Sequenzen mit der in SEQ ID NO: 2, SEQ ID NO: 4, SEQ ID NO: 6, SEQ ID NO: 8, SEQ ID NO: 10, SEQ ID NO: 12 und SEQ ID NO: 14 beschriebenen Struktur, und
b) DNA-Sequenzen, die unter Standardbedingungen mit DNA Sequenzen a) hybridisieren, gebildet wird,
ausgewählt sind. Hierbei handelt es sich um DNA-Sequenzen für Thrombininhibitoren mit bis zu sechs Domänen.

Auch kleinere Proteine, die nur eine dieser beiden Domänen enthalten sind Gegenstand der Erfindung. Weiterhin sind als erfindungsgemäße Proteine auch solche zu verstehen, die mehrere, bevorzugt bis zu 10, solcher Domänen enthalten.

Dabei sind die Mindestanforderungen für eine aktive Einzeldomäne die Aminosäuren (aus SEQ ID NO: 7) ab Position 6 bis 48, 57 bis 101 oder 119 bis 160 oder die Aminosäuren aus SEQ ID NO: 17 ab Position 6 bis 48, 57 bis 101, 120 bis 161, 190 bis 232, 241 bis 285 oder 303 bis 344.

N-terminale und C-terminale Extensionen stören die thrombininhibierende Aktivität nicht. Solche Verlängerungen können z.B. Sequenzen beinhalten wie die Aminosäuren aus SEQ ID NO: 7: 1 bis 5, 49 bis 56, 102 bis 118, 161 bis 170 oder aus SEQ ID NO: 17: 1 bis 5, 49 bis 56, 102 bis 119, 162 bis 189, 233 bis 240, 286 bis 302 oder 345 bis 354.

Diese Aminosäuresequenzen, die sich durch hohe Flexibilität und Hydrophilie auszeichnen, können als Abstandhalter zwischen den einzelnen Domänen eingesetzt werden. Solche Multidomänen-Proteine zeigen eine verlängerte Halbwertszeit, ohne daß die Aktivität der Einzeldomäne wesentlich sinkt.

Durch die Insensitivität des neuen Proteins und seiner Einzeldomänen gegenüber Verlängerungen am N-Terminus und C-Terminus ist es auch möglich, den neuen Thrombininhibitor heterolog mit anderen Proteinen zu verbinden. Solche Proteine sind zum Beispiel Gewebe-Plasminogen-Aktivatoren, Streptokinase, Urokinase, Anti-Thrombin III und aktiviertes Protein C. Ein wichtiges Protein für die Lysetherapie ist tPA, seine Muteine und Derivate. Der Vorteil solcher Fusionsproteine ist eine erhöhte Thrombusspezifität bei gleichzeitiger Verringerung der Wiederverschlußrate.

Beispielsweise wird die Aminosäuresequenz des Gewebeplasminogenaktivators (EP 93619) zwischen Position 1 und 527 mit den Aminosäuren 1 bis 104 des doppelköpfigen Thrombininhibitors verlängert, indem hinter die für tPA 1 bis 157 kodierende Nukleotid-Sequenz die entsprechende Nukleotidsequenz aus SEQ ID NO: 2 über geeignete Restriktionsstellen verknüpft wird.

Umgekehrt ist es aber auch möglich, zum Beispiel die Nukleotidsequenz ab Aminosäure 104 des Doppelkopfinhibitors mit der Sequenz für den Gewebeplasminogenaktivator zu verlängern, indem die Aminosäure 1 des tPA zur Aminosäure 105 des heterotrimeren Proteins wird.

Durch solche Kombinationsproteine wird eine höhere Gerinnselspezifität erreicht, da der Thrombininhibitor selektiv das in den Thrombus eingeschlossene Thrombin bindet und seinen Fusionspartner so an das Gerinnsel heranführt.

Eine alternative Strategie ist das oben beschriebene Fusionsprotein mit einer durch Proteasen spaltbaren Sequenz (z.B. einer Faktor Xa-Schnittstelle (ILE-GLU-GLY-ARG-X)) zu versehen, wodurch nach einer Aktivierung des Gerinnungssystems die Fusionspartner freigesetzt werden, um z.B. ihre fibrinolytische und antikoagulatorische Wirkung auszuüben. Es können mehrere, bevorzugt 2 bis 10 Fusionspartner so zusammengestellt werden.

Solche Proteine, die mehrere dieser Domänen enthalten, werden zweckmäßigerweise durch gentechnische Verfahren hergestellt. Man verknüpft beispielsweise die DNA-Sequenzen die für die Domänen codieren, nach bekannten Verfahren zu einem synthetischen 'Multidomänen-Gen' und exprimiert dieses Gen in an sich bekannter Weise.

Das neue Protein läßt sich aus Raubwanzen der Gattung Rhodnius isolieren. Hierzu werden die Raubwanzen zweckmäßigerweise in einem Puffer bei pH 6 bis 9, vorzugsweise pH 7 bis 8 aufgenommen und mit einem Homogenisator, vorzugsweise einem Mixer, homogenisiert. Anschließend werden die unlöslichen Bestandteile abgetrennt, bevorzugt abzentrifugiert.

Die weitere Reinigung des Proteins kann über chromatographische Methoden, bevorzugt Ionenaustauschchromatographie und/ oder Affinitätschromatographie erfolgen. Besonders bevorzugt ist ein Reinigungsschritt über Thrombin-Affinitätschromatographie.

Die Reinigung des Proteins kann über einen Thrombin-Aktivitätstest verfolgt werden. Hierzu benutzt man zweckmäßigerweise einen optischen Test, bei dem ein chromogenes Substrat, beispielsweise Chromozym T, durch Thrombin umgesetzt wird. Die das neue Protein enthaltenden Fraktionen können bei Zugabe in diesen optischen Test an ihrer Thrombin inhibierenden Wirkung erkannt werden.

Besonders geeignet zur Herstellung des erfindungsgemäßen Proteins sind gentechnische Verfahren.

Hierzu wird in an sich bekannter Weise eine cDNA-Genbank aus der Raubwanze angelegt. Aus dieser Genbank kann das für das erfindungsgemäße Protein kodierende Gen isoliert werden, indem man beispielsweise eine DNA-Probe herstellt, deren Sequenz von der oben beschriebenen N-terminalen Aminosäuresequenz durch Rückübersetzung gemäß dem genetischen Code erhalten wird. Durch Hybridisierung mit dieser DNA-Probe läßt sich das entsprechende Gen auffinden und isolieren.

Für die Herstellung des entsprechenden Gens kann aber auch die Polymerase-Chain-Reaction (PCR)-Technik eingesetzt werden. Beispielsweise läßt sich mit Hilfe eines Primers, dessen Sequenz durch Rückübersetzung aus der oben beschriebenen N-terminalen Aminosäuresequenz erhalten wurde, und eines zweiten Primers, dessen Sequenz komplementär zum 3'-Ende des cDNA-Genfragments ist, bevorzugt mit der Sequenz Poly(dT), das cDNA-Genfragment für das erfindungsgemäße Protein durch PCR-Technik herstellen. Das entsprechende Gen läßt sich auch isolieren, indem man eine Expressionsgenbank von Raubwanzen anlegt und diese mit einem Antikörper der gegen das erfindungsgemäße Protein gerichtet ist, absucht.

Nachdem das entsprechende Gen isoliert worden ist, kann es durch gentechnische Verfahren in Organismen, z.B. in Bakterien, Hefen und anderen eukaryontischen Zellen, mit Hilfe eines Expressionsvektors in an sich bekannter Weise exprimiert werden.

Bevorzugt wird in Prokaryonten, wie z.B. E. coli und mit Hilfe stark exprimierender Vektoren, z.B. unter der Kontrolle des induzierbaren tac-Promotors, wie z.B. in Plasmid pMal-p2 (Protein Fusion and Purification System, "GeneExpress", New England Biolabs) vorhanden, gearbeitet. Dabei kommt es zur periplasmatischen Expression eines Fusionsproteins aus dem Maltose-Bindungsprotein und dem beschriebenen Thrombininhibitor. Der Fusionspartner kann nach Reinigung enzymatisch entfernt werden.

Die generelle Vorgehensweise zur gentechnischen Herstellung eines neuen Proteins bei bekannter Aminosäurepartialsequenz ist in Lehrbüchern der Gentechnologie, beispielsweise E.L. Winnacker, Gene und Klone, Verlag Chemie, Weinheim, 1984, beschrieben. Die experimentellen Bedingungen für die einzelnen Verfahren wie beispielsweise Anlegen einer Genbank, Hybridisierung, Expression eines Gens sind bei T. Maniatis, "Molecular Cloning", Cold Spring Harbor Laboratory, 1990, beschrieben.

Unter Standardbedingungen sind beispielsweise Temperaturen zwischen 42 und 58°C in einer wäßrigen Pufferlösung mit einer Konzentration zwischen 0,1 und 1 x SSC (1 x SSC: 0,15M NaCl, 15nM Natriumcitrat pH 7,2) zu verstehen.

Das erfindungsgemäße Protein wird bevorzugt in Form seiner pharmazeutisch annehmbaren Salze verwendet.

Das neue Protein besitzt blutgerinnungshemmende Eigenschaften. Es kann beispielsweise zur Prophylaxe von Thrombosen oder arteriellen Reokklusionen, zur Behandlung von Thrombosen, zur Konservierung von Blut oder bei der extrakorporalen Zirkulation verwendet werden.

Die neuen Proteine sind wirksame Thrombininhibitoren. Sie können allein oder auch zusammen mit bekannter gerinnungshemmenden Faktoren als Arzneimittel verwendet werden. Als gerinnungshemmende Faktoren werden bevorzugt Thrombininhibitoren, beispielsweise Hirudin, Faktor Xa-Inhibitoren, beispielsweise TAP (Waxman et al., Science 248, 1990, Seite 593-596) oder Plättchen-Aggregationshemmer, beispielsweise Kistrin (Dennis et al., Proc. Natl. Acad. Sci. USA, 87, 1989, Seite 2471-2475) eingesetzt.

Die Erfindung wird durch die folgenden Beispiele weiter veranschaulicht.

### Beispiel 1

### Reinigung des thrombininhibitorischen Proteins aus Raubwanzen

Eine Laborkultur der Raubwanzen (Rhodnius prolixus) wurde bei 28°C und 80 % relativer Luftfeuchtigkeit gehalten. In 30-tägigen Abständen wurden die Raubwanzen dadurch gefüttert, daß sie an Kaninchen saugen konnten. Die Raubwanzen wurden nach Erreichen des letzten Entwicklungsstadiums bei -20°C eingefroren.

25 g Raubwanzen wurden mit 75 ml 20 mM Natriumphosphatpuffer, 150 mM NaCl (pH 7,5) homogenisiert. Das Homogenat wurde 60 Minuten bei 20000 U/min (Sorvall RC-3B, Rotor SS-34) zentrifugiert. Der Niederschlag wurde verworfen.

Die Proteinlösung (Überstand) wurde auf eine Q-Sepharose Säule® (Pharmacia) aufgetragen (60 ml/h), die in 20 mM Natriumphosphatpuffer pH 8,0 äquilibriert worden war (Durchmesser 2,5 cm, Volumen 50 ml).

Es wurde mit 10 Säulenvolumen Äquilibrierungspuffer gewaschen.

Danach wurde ein linearer Gradient von 50 ml 20 mM Natriumphosphat (pH 8,0), nach 50 ml 20 mM Natriumphosphat (pH 8,0), 1 M NaCl angelegt.

Aktive Fraktionen (gemessen durch Thrombininhibition) wurden gesammelt.

Die vereinigten aktiven Fraktionen wurden auf eine Affinitätssäule mit immobilisiertem Thrombin aufgetragen (Durchmesser 1,5 cm, Höhe 6,5 cm, 11,5 ml Säulenvolumen, 60 ml/h). Die Säule wurde gemäß Beispiel 3 hergestellt.

Die Säule wurde mit 20 mM Natriumphosphat pH 7,5 äquilibriert. Nach Auftragen der Proteinlösung wurde die Säule mit 10 Säulenvolumen Äquilibrierungspuffer gewaschen, bis die Absorption bei 280 nm auf Null zurückging.

Danach wurde mit 0,5 M NaCl, 20 mM Natriumphosphatpuffer pH 7,5 gewaschen. Unspezifisch adsorbiertes Material wurde so entfernt.

Spezifisch an Thrombin gebundenes Protein wurde mit 0,1 M Glycin, 0,5 M NaCl pH 2,8 eluiert. Die Säule wurde anschließend sofort wieder mittels Phosphatpuffer auf pH 7,5 eingestellt.

Die einzelnen Fraktionen wurden mit 0,1 M NaOH neutralisiert und auf ihre inhibitorische Wirkung gegen Thrombin untersucht.

Die durch Glycin/NaCl-Puffer pH 2,8 eluierten Fraktionen besaßen thrombininhibierende Wirkung.

Die gesammelten aktiven Fraktionen wurden, nachdem sie neutralisiert worden waren, mit Wasser verdünnt (1:10) und auf eine Mono-Q-Säule® gegeben (Pharmacia, 1 ml Säulenvolumen).

Die Säule wurde äquilibriert mit 20 mM Natriumphosphatpuffer pH 7,5, 150 mM NaCl (Puffer A). Es wurde mit Puffer A gewaschen bis die Absorption auf Null zurückging (10 Minuten). Dann wurde innerhalb von 50 Minuten auf 20 mM Natriumphosphat, pH 7,5, 800 mM NaCl (Puffer B) gewechselt (Fluß 0,5 ml/min).

Thrombin hemmende Fraktionen wurden gesammelt.

Die gesammelten Fraktionen wurden an einer RP 318® (Biorad) HPLC-Chromatographiesäule weiter gereinigt. Die Säule wurde äquilibriert mit 0,1 Gew.% Trifluoressigsäure (TFA) in dest. Wasser. Die vereinigten aktiven Fraktionen wurden auf die Säule aufgetragen. Mit einem Gradienten nach 0,1 Gew.-% TFA, 100 % Acetonitril während einer Stunde und einer Flußrate von 1 ml/min wurde die Säule eluiert. Die Absorption wurde bei 280 nm bestimmt. Es wurden 0,5 ml Fraktionen gesammelt. Die gesammelten Fraktionen wurden zur Trockene einkonzentriert und in Phosphat gepufferter Salzlösung (PBS) (0,8 g/1 NaCl; 0,2 g/l HCl; 0,144 g/l Natriumphosphat; 0,2 g/l Kaliumphosphat, pH 7,5) aufgenommen sowie die inhibitorische Aktivität bestimmt.

Die Proteinbestimmung erfolgte nach der Vorschrift von Bradford (Anal. Biochem., 72, 248-254 (1976)). Als Standardprotein diente Rinderserumalbumin (Boehringer Mannheim).

### Beispiel 2

### Bestimmung der Hemmung von Thrombin durch den Inhibitor

Thrombin (Boehringer Mannheim) wurde zu einer Endkonzentration von 25 mU/ml in Phosphat gepufferter Salzlösung (PBS) (0,8 g/l NaCl; 0,2 g/l HCl; 0,144 g/l Natriumphosphat; 0,2 g/l Kaliumphosphat, pH 7,5) gelöst.

Chromozym TH (Boehringer Mannheim) wurde in 20 ml H₂O/Flasche gelöst.

50 µl Thrombinlösung und 100 µl Chromozym sowie 25 µl Probe oder Puffer wurden in die Näpfe einer Mikrotiterplatte gegeben. Sofort danach wurde zur Zeit 0 und nach 30 Minuten bei 37°C die Absorption bei 405 nm gemessen.

Bei starker Eigenfarbe der Probe wurde eine weitere Kontrolle ohne Thrombin wie oben behandelt.

Durch die Aktivität des Thrombins wird aus dem chromogenen Farbsubstrat ein bei 405 nm absorbierender Farbstoff freigesetzt. Die Hemmung des Thrombins durch einen Thrombininhibitor ist an einer geringeren Absorptionszunahme bei 405 nm erkennbar und wurde mit Hilfe einer Eichkurve quantifieziert.

### Beispiel 3

### Herstellung einer Affinitätssäule mit Thrombin als Ligand

a) Kopplung:
   2 g CNBr aktivierte Sepharose (Pharmacia) wurden mit 200 ml 1 mM HCl auf einer Nutsche gewaschen. Das Gel wurde in 100 mM NaHCO₃, 500 mM NaCl pH 8,3 aufgenommen und sofort mit 10000 Units Thrombin (Sigma) in 100 mM NaHCO₃, 500 mM NaCl, pH 8,3 gemischt.
   Die Lösung wurde 24 Stunden bei 4°C vorsichtig geschüttelt.
b) Blockierung:
   Das Gelmaterial wurde nach Absetzen mit 100 mM NaHCO₃, 500 mM NaCl, pH 8,3 gewaschen. Die Sepharose wurde dann mit 100 mM NaHCO₃, 500 mM NaCl, 1 M Ethanolamin pH 8,3 für 2 Stunden inkubiert.
c) Vorbereitung:
   Zur Entfernung des ungebundenen Thrombin wird das Gelmaterial vor Gebrauch noch einmal in der Säule mit 20 Säulenvolumen PBS pH 7,4 gewaschen.

### Beispiel 4

### Bestimmung des Molekulargewichts durch Molekularsiebchromatographie.

Bei einer Flußgeschwindigkeit von 1 ml/min wurde durch Mono-Q®-Chromatographie gereinigtes Material in 20 mM Natriumphosphat, 150 mM NaCl, pH 7,5 auf einer Molekularsiebsäule des Typs TSK® (Pharmacia, Spherogel TSK 3000® SW, 7,5 mm Durchmesser, 60 cm Höhe) getrennt.

Ebenso wurde mit den Eichproteinen verfahren (Serumalbumin MW 67000 Da, Ovalbumin MW 45000 Da, Chymotrypsinogen A MW 25000 Da).

Der Logarithmus des Molekulargewichts der Eichproteine wurde gegen deren Elutionszeit in einem Diagramm aufgetragen.

In den fraktionierten Eluaten der Probe wurde die Thrombininhibition bestimmt.

Die Elutionszeit des Inhibitors ergab am Schnittpunkt mit der Eichgeraden den Logarithmus des gesuchten Molekulargewichts.

Durch diese Bestimmung wurde ein Molekulargewicht zwischen 20000 und 24000 Dalton bestimmt.

### Beispiel 5

### Bestimmung des Molekulargewichts durch Tricine-SDS-Polyacrylamid Gel Elektrophorese.

(Literatur: Analytical Biochemistry, 166, 368 - 379 (1987) Tricine-Sodium Dodecyl Sulfate-Polyacrylamide Gel Electrophoresis for the Separation of Proteins in the range from 1 - 1000 kDa, Schägger, H. and von Jagow, G.)

Die Gelelektrophorese wurde entsprechend der Literaturvorschrift bei 20 mA und 1400 V, 30 Watt, durchgeführt.

Das nach dieser Methode bestimmte Molekulargewicht betrug 12000 ± 2000 Dalton.

Als Eichproteine dienten (Intaktes Myoglobin 17,2 kDa, Bromcyanpeptid Myoglobin I+II 14,6 kDa, Bromcyanpeptid Myoglobin I 8,2 kDa, Bromcyanpeptid Myoglobin II 6,4 kDa, Bromcyanpeptid Myoglobin III 2,6 kDa, Myoglobin 1-14)

### Beispiel 6

### Sequenzbestimmung des Inhibitors Reduktion und Carboxymethylierung.

2,8 ml Proteinlösung (0,029 mg/ml) wurden mit 0,28 ml Puffer (1 M Tris/HCl, 0,5 M Guanidinhydrochlorid, pH 8,6) gemischt. Danach wurden 0,116 ml Dithiothreitol (DTT, 10 mg/ml) zugegeben und 10 Minuten bei 37°C inkubiert. Danach wurde nach Zugabe von 0,185 ml Jodacetamid (10 mg/ml) für 90 Minuten bei 37°C inkubiert. Die Reaktion wurde mit 0,073 ml DTT wie oben beendet.

Das Protein wurde durch erneute reversed phase HPLC an RP 318® gereinigt. Das Gemisch wurde auf eine Endkonzentration von 0,1 Gew.% Trifluoressigsäure (TFA) eingestellt und in einem HPLC System von Hewlett Packard (HP 1090 Liquid Chromatograph) getrennt. Mit Lösungsmittel A (0,1 Gew.-% TFA, 100 % H₂O) wurde 5 Minuten gewaschen. Dann wurde der Anteil von Lösungsmittel B (90 % Acetonitril, 10 % H2O, 0,1 Gew.% TFA) im Verlauf von 120 Minuten auf 50 % angehoben. Die Absorption des Eluats bei 214 und 280 nm wurde gemessen. Absorbierende Fraktionen wurden gesammelt. Das Protein wurde durch SDS-Gelelektrophorese identifiziert und einer Sequenzanalyse auf einem Applied Biosystems 477 A Protein Sequencer nach Vorschrift des Geräteherstellers unterworfen.

Folgende aminoterminale Sequenz wurde erhalten (SEQ ID NO: 1):

### Beispiel 7

### Bestimmung des isoelektrischen Punktes durch isoelektrische Fokussierung

Die Bestimmung wurde mit einem LKB Multiphor 2117 (Horizontalsystem) und einem LKB powersupply 2103 durchgeführt. Es wurden Fertiggele eingesetzt (Pharmacia Ampholine PAGplate pH 3,5 - 9,5). Als Standardproteine wurden Amyloglucosidase pH 3,5; Soyabean Trypsin Inhibitor, pH 4,55; β-Lactoglobulin A, pH 5,2; Bovine carbonic anhydratase, pH 5,85; Human carbonic anhydratase, pH 6,55; Horse myoglobin, pH 6,85 und 7,35; Lentil Lectin, pH 8,15, 8,45, 8,65 und Trypsinogen, pH 9,3 eingesetzt.

Die Bedingungen der Fokussierung: 1500 Volt, 30 Watt.
- Puffer:: Anode 1M Phosphorsäure
Kathode 1M Natronlauge

Die Platten wurden 30 Minuten zur Ausbildung eines pH-Gradienten vorfokussiert. Die Proben wurden auf Filterplättchen aufgetragen, die auf dem Gel lagen. Für 30 Minuten wurde weiterfokussiert, die Filterplättchen abgenommen, und nach weiteren 30 Minuten wurde die Fokussierung beendet. Die Gele wurden sofort in 2 mm Scheiben geschnitten und in dest. Wasser überführt. Über Nacht eluierte das Protein aus den Gelscheiben. Durch einen Thrombininhibitionstest wurde die Lage des Thrombininhibitors bestimmt. Der pH-Wert kann auch direkt durch eine pH-Elektrode bestimmt werden. Hirudin, als Vergleichssubstanz hatte einen isoelektrischen Punkt von pH 3,5 und niedriger. Der neue Inhibitor hatte einen isoelektrischen Punkt von pH 4,2 ± 0,5.

### Beispiel 8

Herstellung einer DNA-Sequenz, die für ein thrombininhibitorisches Protein codiert.
a) Isolierung von RNA und Herstellung einer cDNA-Bank
   Gesamt-RNA aus ganzen Tieren der Spezies Rhodnius prolixus wurde durch Aufschluß in Guanidiniumthiocyanat gewonnen. Dabei wurde mit Materialien und nach Anleitung des "RNA Isolation Kit" der Firma Stratagene, La Jolla, CA, USA (Catalog Nr. 200345) gearbeitet. Die polyadenylierte messenger RNA wurde aus der o.a. Gesamt-RNA durch oligo(dT)-Affinitätsseparation selektiert. Dieses Verfahren wurde mit Materialien und nach Anleitung des "PolyATtract mRNA Isolation System" der Firma Promega, Madison, WI, USA (Catalog Nr. Z5200) durchgeführt.
   Aus polyadenylierter messenger RNA wurde mit Materialien und nach Anleitung des "ZAP-cDNA Synthesis Kit" der Firma Stratagene, La Jolla, CA, USA (Catalog Nr. 200400) cDNA synthetisiert, die dann mit Materialien und nach Anleitung des "Uni-ZAP XR GigapackII Cloning Kit" der Firma Stratagene,
   La Jolla, CA, USA (Catalog Nr. 237611) in Lambda Phagen verpackt wurde.
b) Herstellung von Oligonukleotidproben für die PCR
   Zur Klonierung von cDNA-Fragmenten mit Hilfe der Polymerase-Kettenreaktion (PCR, s. "Molecular Coning", 2nd edition (1989), Sambrook, J. et al., CSH-Press, Seite 14.1 ff.) wurde von Peptiden der in Beispiel 6 beschriebenen aminoterminalen Sequenz (SEQ ID NO: 1) ausgegangen.
   Unter Zugrundelegung des genetischen Codes läßt sich aus der Peptidsequenz SEQ ID NO: 18: die Nukleinsäuresequenz SEQ ID NO: 19: des kodierenden DNA-Strages herleiten. Wegen der bekannten Degeneration des genetischen Codes sind an manchen Positionen mehrere Nukleotide (N: A, C, G, T; Y: C, T;) einsetzbar. Damit ergibt sich während der Oligonukleotid-Synthese eine Komplexität von 512 verschiedenen Oligonukleotiden.
   Die Synthesen wurden mit einem Applied Biosystems Typ 360A DNA-Synthesizer durchgeführt. Die Oligonukleotide wurden nach Entfernung der Schutzgruppen gelelektrophoretisch über ein Acrylamid/Harnstoff-Gel gereinigt.
c) Herstellung von DNA-templates für die PCR
   5 µg Gesamt-RNA oder 1 µg Poly(A)⁺-RNA aus der unter a) aufgeführten RNA-Präparation wurden mit dem Oligonukleotid A-B-T₁₈, (SEQ ID NO: 20): und mit Hilfe des Enzyms Reverse Transkriptase in einzelsträngige cDNA (1°cDNA) übersetzt. Dabei wurde mit Materialien und nach Anleitung des "SuperScript Preamplification System" der Firma Gibco BRL, Eggenstein, Deutschland (Catalog Nr. 8089SA) gearbeitet. Nach Beendigung der Reaktion wurden die niedermolekularen Bestandteile über Biospin-30-Säulen der Firma BioRad, Richmond, CA, USA (Catalog Nr. 732-6006) abgetrennt.
d) PCR und Klonierung
   Die Polymerase-Kettenreaktion wurde nach bekannten Protokollen durchgeführt (s. "Molekular Cloning", 2nd edition (1989), Sambrook, J. et al., CSH-Press, Seite 14.1 ff.). Dazu wurde ein "DNA Thermal Cycler" der Firma Perkin Elmer benutzt. Dabei wurde das Prinzip der "verschachtelten Primer" nach Frohmann, M.A. et al. (Proc. Natl. Acad. Sci., USA (1988) 85, 8998-9002) modifiziert angewendet.
   Im einzelnen wurde die 1°cDNA aus c) mit den Oligonukleotiden SEQ ID NO: 19, s.o. und SEQ ID NO: 21 (aus A-B-T₁₈): amplifiziert. Die PCR-Produkte wurden gelelektrophoretisch aufgetrennt und größenfraktioniert. Separierte Agarosescheibchen mit DNA-Fragmenten steigender Molmasse wurden dann in eine zweite PCR mit Oligonukleotid SEQ ID NO: 19, s.o. und SEQ ID NO: 22 (aus A-B-T₁₈): eingesetzt. Die solchermaßen selektierten PCR-Produkte wurden ebenfalls gelelektrophoretisch aufgetrennt und nach Standardmethoden eluiert. Nach Subklonierung in die EcoRV-Schnittstelle des Vektors pBluescriptKS und Vermehrung des Plasmides in E. coli DH5alpha ergab die Sequenzanalyse eines Klones mit der SEQ ID NO: 2 ein offenes Leseraster von 103 Aminosäuren mit der SEQ ID NO: 3, das das in SEQ ID NO: 1 beschriebene Peptid beinhaltet. Der Klon wurde daraufhin pRPTI genannt.
e) Screening der cDNA-Bank
   1x10⁶ rekombinante Phagen der Rhodnius prolixus cDNA-Bank wurden einem Screening mit einer SEQ ID NO: 2 entsprechenden Probe unterzogen. Dabei wurde nach bekannten Protokollen gearbeitet (s. "Molekular Cloning", 2nd edition (1989), Sambrook, J. et al., CSH-Press). Die position Klone wurden sequenzanalysiert und sind als SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 aufgeführt.
f) Heterologe Expression des Thrombininhibitors
   Zur Herstellung des rekombinanten Throminhibitors wurde zunächst die cDNA Sequenz SEQ ID NO: 2 kodierend für die Aminosäuresequenz SEQ ID NO: 3 von Position 1 bis 103 mit Hilfe beschriebener Methoden PCR-amplifiziert und mit passenden Enden in die XmnI und BamHI Schnittstelle des bakteriellen Expressionsvektors pMAL-p2 (Protein Fusion and Purification System, GeneExpress, New England Biolabs Nr. 800) kloniert. Diese Klonierung führt zu einer Fusion des bakteriellen Maltose-Bindungsproteins mit dem beschriebenen Thrombininhibitor im gleichen Leseraster. E. coli DH5alpha Zellen wurden mit dem entstandenen Plasmid transformiert und das rekombinante Fusionsprotein wurde nach Angaben des Herstellers exprimiert und gereinigt. Unter Ausnutzung einer Faktor Xa-Schnittstelle an der Fusionsstelle kann der Thrombininhibitor vom Fusionspartner enzymatisch getrennt werden. Die Ausbeute des in E. coli periplasmatisch exprimierten Thrombininhibitors liegt bei 6000 Units/L Kultur (Standard: National Institute of Health).

## Patentansprüche

1. Protein mit thrombininhibitorischer Wirkung aus Raubwanzen der Gattung Rhodnius, mit einer Aminosäuresequenz, die aus der Gruppe, die von SEQ ID NO: 3, SEQ ID NO: 5, SEQ ID NO: 7, SEQ ID NO: 9, SEQ ID NO: 11, SEQ ID NO; 13 und SEQ ID NO: 15 gebildet wird, ausgewählt ist.

2. Protein mit thrombininhibitorischer Wirkung, enthaltend eine oder mehrere Domänen mit der in SEQ ID NO: 3 angegebenen Aminosäuresequenz.

3. DNA-Sequenzen, die für ein Protein gemäß Anspruch 1 codieren.

4. DNA-Sequenzen, die für Proteine mit thrombininhibitorischer Wirkung codieren, und die aus der Gruppe, die von
a) DNA-Sequenzen mit der in SEQ ID NO: 2, SEQ ID NO: 4, SEQ ID NO: 6, SEQ ID NO: 8, SEQ ID NO: 10, SEQ ID NO: 12 und SEQ ID NO: 14 beschriebenen Struktur, und
b) DNA-Sequenzen, die unter Standardbedingungen mit DNA Sequenzen a) hybridisieren, gebildet wird,
ausgewählt sind.

5. Expressionsvektor, der eine DNA-Sequenz gemäß Anspruch 3 oder 4 enthält.

6. Protein gemäß Anspruch 1 oder 2 zur Verwendung für die Bekämpfung von Krankheiten.

7. Arzneimittel, enthaltend ein oder mehrere Proteine, die von DNA-Sequenzen gemäß Anspruch 4 codiert werden, und einen weiteren gerinnungshemmenden Faktor.

## Claims

1. A protein with thrombin-inhibitory action from assassin bugs of the genus Rhodnius, having an amino-acid sequence which is selected from the group formed by SEQ ID NO: 3, SEQ ID NO: 5, SEQ ID NO: 7, SEQ ID NO: 9, SEQ ID NO: 11, SEQ ID NO: 13 and SEQ ID NO: 15.

2. A protein with thrombin-inhibitory action, containing one or more domains with the amino-acid sequence indicated in SEQ ID NO: 3.

3. A DNA sequence which codes for a protein as claimed in claim 1.

4. A DNA sequence which codes for a protein with thrombin-inhibitory action and which is selected from the group formed by
a) DNA sequences with the structure described in SEQ ID NO: 2, SEQ ID NO: 4, SEQ ID NO: 6, SEQ ID NO: 8, SEQ ID NO: 10, SEQ ID NO: 12 and SEQ ID NO: 14, and
b) DNA sequences which hybridize under standard conditions with DNA sequences a).

5. An expression vector which contains a DNA sequence as claimed in claim 3 or 4.

6. A protein as claimed in claim 1 or 2 for use for controlling diseases.

7. A drug containing one or more proteins encoded by DNA sequences as claimed in claim 4, and another anticoagulant factor.

## Revendications

1. Protéine à activité inhibitrice de la thrombine provenant de punaises hématophages du genre Rhodius avec une séquence d'aminoacides qui est choisie dans le groupe formé par SEQ ID NO: 3, SEQ ID NO: 5, SEQ ID NO: 7, SEQ ID NO: 9, SEQ ID NO: 11, SEQ ID NO: 13 et SEQ ID NO: 15.

2. Protéine à activité inhibitrice de la thrombine, contenant un ou plusieurs domaines avec la séquence d'aminoacides indiquée dans SEQ ID NO: 3.

3. Séquences d'ADN qui codent pour une protéine suivant la revendication 1.

4. Séquences d'ADN qui codent pour des protéines à activité inhibitrice de la thrombine et qui sont choisies dans le groupe formé par
a) des séquences d'ADN avec la structure décrite dans SEQ ID NO: 2, SEQ ID NO: 4, SEQ ID NO: 6, SEQ ID NO: 8, SEQ ID NO: 10, SEQ ID NO: 12 et SEQ ID NO: 14, et
b) les séquences d'ADN qui s'hydrident dans les conditions normales ou standard avec des séquences d'ADN a).

5. Vecteur d'expression qui contient une séquence d'ADN suivant la revendication 3 ou 4.

6. Protéine suivant la revendication 1 ou 2, à utiliser pour lutter contre des maladies.

7. Médicaments qui contiennent une ou plusieurs protéines qui sont codées par des séquences d'ADN suivant la revendication 4, et un autre facteur inhibant la coagulation.
